# EUROPEAN PATENT APPLICATION

(11) **EP 0 769 503 A2**
(43) Date of publication of application: **23.04.1997**
(21) Application number: 96402220.6
(22) Date of filing: 16.10.1996
(51) Int. Cl.: C08B 37/10, A61L 33/00

(54) **A heparin complex and medical device having such substance**

(30) Priority: 17.10.1995 JP 268707/95
(71) Applicant: TERUMO KABUSHIKI KAISHA, Tokyo (JP)
(72) Inventor: Motomura, Tadahiro, c/o Terumo K.K., Ashigarakami-gun, Kanagawa (JP); Anzai, Takao, c/o Terumo K.K., Ashigarakami-gun, Kanagawa (JP); Onishi, Makoto, c/o Terumo K.K., Ashigarakami-gun, Kanagawa (JP)
(74) Representative: Gillard, Marie-Louise

(57) **Abstract**

A heparin complex having anticoagulation activity and being insoluble in blood is provided. The preferred complex comprises a cationic alkyldimethylammonium represented by formula (1): 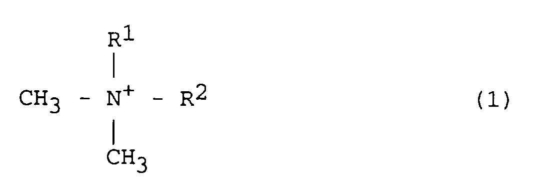 wherein R¹ and R² are independently an alkyl or alkenyl group, each group is C₁₂-C₂₄ when R¹ and R² are the same, the sum of the carbon atoms are 26-38 when R¹ and R² are different; and heparin. In this complex, reduction in the anticoagulation activity of the heparin by the formation of the complex is minimized, and ionic bonding of the heparin is quite stable. Dissolution of the cationic alkyldimethylammonium from the coating is also minimized. A medical device coated with such substance is also provided.

## Description

### BACKGROUND OF THE INVENTION

This invention relates to a novel heparin complex and a medical device having such complex on its surface.

When blood is brought into contact with a foreign substance other than cells on the inner surface of a blood vessel, plasma proteins and platelets start to adhere to the foreign substance, and blood coagulation is initiated resulting in the formation of thrombus. If such thrombus formation may occur during use of a medical device such as catheter, blood circuit, dialyzer or oxygenator, there is a fair risk that the thrombosis may cause blockage of the blood flow and complications such as sepsis. To avoid such unfavorable formation of the thrombus on the blood-contacting surface of the medical device, an anticoagulant is often added to the blood to prevent blood coagulation, and the blood-contacting surface of the medical device is often coated with an antithrombotic substance to prevent thrombus formation on the blood-contacting surface. Investigation of antithrombotic materials that are free from thrombus formation are intensively carried out by various means.

Materials having heparin immobilized on their surface are typical antithrombotic materials. Various antithrombotic materials of such type have been developed in the expectation that formation of a heparin thin layer on the blood-contacting surface should be particularly effective in avoiding thrombus formation. Among such heparin immobilization methods, those wherein the immobilization is attained by ionic bond have been investigated due to the operational convenience of such immobilization, and various reports have been disclosed after the report by Vincent Gott of the heparin immobilization method wherein the blood-contacting surface is treated with graphite, benzalkonium chloride is adsorbed on the thus treated surface, and the heparin is immobilized on the surface by utilizing the ionic bond between the benzalkonium chloride and the heparin. Other methods that have been disclosed include the method wherein the blood-contacting surface is coated with a complex of alkylbenzyl-methylammonium salt and heparin (Japanese Patent Publication No. 2(1990)-36267); and the method wherein heparin is immobilized by ionic bonding to the substrate having a tertiary amine vinyl compound graft-polymerized thereon (Japanese Patent Publication No. 55(1980)-38964). The antithrombotic materials produced by such methods, however, exhibited dissolution of heparin from the surface where they have been immobilized into body fluids such as blood as soon as the surface was brought into contact with the body fluid, and the results were loss of almost all of the immobilized heparin in a relatively short while Such materials also suffered from hemolysis of the blood due to cationic substances such as benzalkonium chloride that had dissolved into the blood simultaneously with the heparin *(J. Biomed. Mater. Res.* vol. 4, 549, 1970). Heparin also experienced a substantial loss of its anticoagulation activity by the formation of a complex with such substances as benzalkonium chloride.

Commercially available benzalkonium chloride is a mixture of salts having different alkyl groups represented by formula (2): 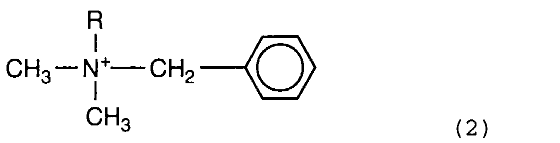 wherein R is a C₈ - C₁₈ alkyl group. The complex of benzalkonium chloride and heparin exhibits high solubilit in water, and when a medical device is coated with such complex, dissolution into the blood of heparin is likely to occur.

USP 4349467 describes a salt of the following compounds represented by formula (3) : 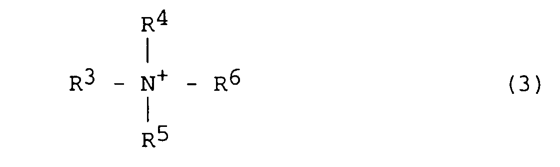 where R³ is a C₁₂ - C₁₈ alkyl radical and R⁴ to R⁶ is any one selected from the group consisting of hydrogen and C₁-C₆ alkyl group. The complex of a salt of the compound of formula (3) and heparin is more soluble in water than the heparin complex of the present invention described below.

Stearylbenzyldimethylammonium salt is also a salt of a compound represented by formula (2), and in this case, R in formula (2) is a C₁₈ alkyl group. Japanese Patent Publication No. 2(1990)-36267 as mentioned above utilizes a complex of stearylbenzyldimethylammonium with heparin. This complex also exhibits rapid dissolution into the blood, and the surface where it had been immobilized experiences a considerable loss of heparin activity (anti-FXa activity).

### SUMMARY OF THE INVENTION

In view of the situation as described above, an object of the present invention is to provide a novel heparin complex and a medical device having such complex on its surface wherein reduction in the anticoagulation activity of heparin by the formation of the complex is minimized, and the ionic bond of the heparin is quite stable, and wherein dissolution of the cationic alkyldimethylammonium substance into blood or physiological saline is minimized.

Another object of the present invention is to provide a medical device having such complex on its surface.

According to the present invention, there is provided:
(1) a heparin complex comprising a cationic alkylammonium and a heparin which does not substantially dissolve into the blood,
(2) a heparin complex comprising a cationic alkyldimethylammonium represented by formula (1): 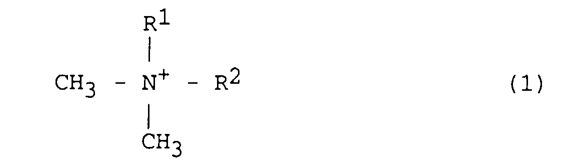 wherein R¹ and R² are independently an alkyl or an alkenyl group, when R¹ and R² are the same, R¹ and R² represent any alkyl or alkenyl group containing 12 to 24 carbon atoms, and when R¹ and R² are not the same, one of R¹ and R² represents any alkyl or alkenyl group containing at least 10 carbon atoms and the sum of the carbon atoms in R¹ and R² is 26-38, preferably an alkyl group containing 12, 14, 16 and 18 carbon atoms; and heparin.
(3) a substance having anticoagulation activity comprising a complex of a cationic alkyldimethylammonium represented by above described formula (1):
   wherein R¹ and R² are independently an alkyl group containing at least 18 carbon atoms, preferably 18 carbon atoms; and heparin,
(4) the heparin complex having a solubility of at least 0.1% by weight in terms of concentration in an organic solvent having a dielectric constant of 18.3 or less than 18.3; and a solubility of up to 0.1% by weight in an organic solvent having a dielectric constant of more than 18.3.
(5) the substance having anticoagulation activity comprising a complex of a cationic alkyldimethylammonium of formula (1) as defined in (3), said substance having a solubility of at least 0.1% by weight in terms of concentration in an organic solvent having a dielectric constant of 17.1 or less than 17.1; and a solubility of up to 0.1% by weight in an organic solvent having a dielectric constant of 17.1 or more than 17.1.
(6) a heparin complex comprising a distearyldimethylammonium and heparin, and
(7) a medical device which has such a substance having anticoagulation activity or a heparin complex described above on at least a part of a surface thereof.

### DETAILED DESCRIPTION OF THE INVENTION

The anticoagulant substance of the present invention is a heparin complex comprising a cationic alkylammonium and heparin and does not substantially dissolve in blood. The phrase "does not substantially dissolve in" means in this specification "a solubility of up to 0.1% by weight in a solvent". The alkyl group may be an alkyl group having 1 to 24 carbon atoms. The complex has a solubility of at least 0.1% by weight in terms of concentration in an organic solvent having a dielectric constant of 18.3 or less than 18.3; and a solubility of up to 0.1% by weight in an organic solvent having a dielectric constant of more than 18.3 is more preferable. The solvent having a dielectric constant of 18.3 or less than 18.3 is exemplified isopropanol, n-butanol, methylenechloride and tetrahydrofurane(THF). The solvent having a dielectric constant of more than 18.3 is exemplified methanol and ethanol. The slovent described above may be a mixture of compounds.

Another anticoagulant substance of the present invention is a heparin complex comprising a cationic alkyldimethylammonium represented by formula (1): 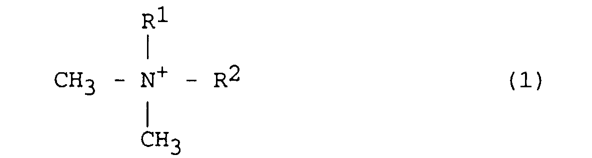 wherein R¹ and R² are independently an alkyl or an alkenyl group, when R¹ and R² are the same, R¹ and R² represent any alkyl or alkenyl group containing 12 to 24 carbon atoms and, when R¹ and R² are not the same, one of R¹ and R² represents any alkyl or alkenyl group containing at least 10 carbon atoms and the sum of the carbon atoms in R¹ and R² is 26-38; and heparin.
The alkyl or alkenyl group may be straight, branched chains or their structural isomers.

Preferable examples of the R¹ and R² group, when they are the same, are straight chains of C₁₂H₂₅, C₁₄H₂₉, C₁₆H_{33,} C₁₈H₃₇, C₂₀H₄₁, C₂₂H₄₄, C₂₄H₄₉**,** 17-octadecenyl and 3,3-dimethyl hexadecyl and the like. Preferable examples of the R¹ and R² group, when they are different, are straight chains of the combination of C₁₂H₂₅ and C₁₄H₂₉, C₁₆H₃₃ and C₁₄H₂₉, C₁₈H₃₇ and C₁₆H_{33,} C₁₂H₂₅ and C₁₆H_{33,} C₁₂H₂₅ and C₂₀H₄₁, C₁₈H₃₇ and C₂₀H₄₁, and the like. Among them, R¹ and R² groups having straight chains of C₁₂H_{25,} and C₁₄H_{29,} C₁₆H₃₃ and C₁₈H₃₇ are more preferable. The heparin complexes of C₁₂, C₁₄, C₁₆ and C₁₈ dialkyldimethyl ammonium hardly activate a complement C3a in blood. The heparin complex of the present invention may inhibit the activation of a complement such as C3a.

The heparin which may be used in the present invention is a commercially available heparin having a molecular weight in the range of from 7 x 10³ to 25 x 10³. A preferable example of such heparin is the one manufactured by Scientific Protein Lab.

The heparin complex of the present invention may be prepared by adding the alkylammonium of formula (1) into an aqueous solution of heparin and recovering the precipitate so formed.

The heparin complex of the present invention is highly hydrophobic and hardly soluble in water, and hardly soluble from the surface of the medical device where it has been applied into the physiological saline or the blood that is in contact with the surface, and in particular, when the cationic alkyldimethylammonium having dialkyl of C₁₂H₂₅, C₁₄H₂₉, C₁₆H₃₃ or C₁₈H₃₇ is employed. As a consequence, the surface of the medical device undergoes little reduction in heparin activity (anti-FXa activity) upon priming with the physiological saline or contact with the blood since the heparin complex does not dissolve from the surface of the medical device.

Such an advantage of the heparin complex of the present invention is believed to have been realized by the relatively high hydrophobicity of the substance owing to the two long-chain alkyl moieties of the cationic alkyldimethylammonium salt in the substance. When compared to stearylbenzylammonium salt-heparin complex which is a counter-example, the heparin complex of the present invention exhibits higher hydrophobicity, and hence, reduced solubility in water, as demonstrated by the difference in solubility in various solvents having different dielectric constants.

Owing to the improved hydrophobicity of the heparin complex of the present invention, lipophilicity of the heparin is attained with a small amount of cationic alkylammonium salt. In the case of the counter-example dimetyl stearylbenzylammonium-heparin complex, the amount of the ammonium bonded to the heparin is as high as about 70% when evaluated by elemental analysis by oxidation through thermal decomposition (wherein CO₂, N₂, H₂O and SO₂ produced by combustion in oxygen atmosphere at a high temperature are analyzed). In contrast, in the stearyl dimethylammonium-heparin complex of the present invention, the amount of the ammonium salt is 55% by weight or less.

This demonstrates that the lipophilicity of the heparin has been attained with a smaller amount of ammonium salt, which in turn means a smaller number of the blocked sulfate moiety which is the site of the heparin activation. The high anticoagulation activity of the substance of the present invention is thus attained.

The ammonium of the present invention such as distearyldimethylammonium has excellent safety as demonstrated by low toxicity upon oral administration compared to the conventional compound, benzalkonium chloride.

### Reference Example: Toxicity upon oral administration to rat

| Compound | LD₅₀,mg/kg |
|---|---|
| Benzalkonium dimethylammonium (C₈-C₁₈) chloride | 445 |
| Stearylbenzyldimethylammonium chloride | 1250 |
| Laurylbenzyldimethylammonium chloride | >500 |
| Trimethyloctadecylammonium chloride | 1000 |
| Trimethylhexadecylammonium chloride | 250 - 300 |
| Distearyldimethylammonium chloride | 11300 |

The medical devices that may be coated with the heparin complex of the present invention include catheters blood circuits, dialyzers, artificial (supplementary) hearts, oxygenators,dwelling needles, artificial kidneys, stents , and the like. The material which constitutes such medical devices are not limited to any particular polymers, and may comprise vinyl chloride, polyurethane, polyamide, polyester, polycarbonate, polyolefin, a modification product thereof, carbon-based material, and the like. The surface of the medical device may be treated by hydrophobic pretreatment, before application of the complex of the present invention.

Preferably, after the medical devices which are coated with the heparin complex of the present invention, have been contacted with blood for 180 minutes, the medical devices of the present invention have residual heparin activity of 0.2x10⁻² U/cm⁻² or more.

The methods for coating the heparin complex of the present invention on the surface of the medical device are not limited to any particular methods, and the medical device may be coated by a coating method such as coating, spin coating, dipping, or spraying. The complex of the present invention may dissolve or disperse an organic solvent and may be coated on the surface of a medical device. The solvent may be one solvent or a mixture of solvents.

The present invention is further described by referring to the following Examples and Comparative Examples.

### Example

To a solution of 4.3 g of heparin (manufactured by Scientific Protein Lab.) in 50 ml of filtered water by reverse osmosis (RO water) was added dropwise a solution of 10 g of distearyldimethylammonium salt (manufactured by Tokyo Kasei) in 700 ml of RO water with stirring. The resulting precipitate was recovered by filtration, and suspended in 500 ml of RO water with stirring to remove the excessive distearyldimethylammonium salt and obtain the distearyldimethylammonium-heparin complex (DC18) which is a heparin complex of the present invention. Elemental analysis of the heparin complex DC18 revealed that the substance contained 54% of distearyldimethylammonium salt bonded thereto.

Further, the same procedure described above was repeated to produce the heparin complex of the following cationic ammonium;
dimethyl didodecyl ammonium (DC12),
dimethyl ditetradecyl ammonium (DC14),
dimethyl dihexadecyl ammonium (DC16),
dimethyl dieicosanyl ammonium (DC20),
dimethyl didocosanyl ammonium (DC22),
dimethyl ditetraeicosanyl ammonium (DC24),
dimethyl di(17-octadecenyl)ammonium (DC18d),
dimethyl di(3,3-dimethylhexadecyl) ammonium (DC18mm),
dimethyl dodecyl tetradecyl ammonium (C12C14),
dimethyl hexadecyl tetradecyl ammonium (C16C14),
dimethyl hexadecyl stearyl ammonium (C18C16),
dimethyl dodecyl hexadecyl ammonium (C12C16),
dimethyl dodecyl eicosanyl ammonium (C12C20),
dimethyl stearyl eicosanyl ammonium (C18C20),

### Comparative Example

The procedure of Example 1 was repeated to produce stearylbenzylammonium salt-heparin complex (SBH) described in Japanese Patent Publication No. 2(1990)-36267 for comparison purposes. Elemental analysis of the anticoagulant substance revealed that the substance contained 63% of stearylbenzylammonium salt bonded thereto.

The heparin complexes of ;
benzalkonium ammonium (BZH)
cetylpyridinium ammonium (CPH),
trimethyl dodecyl ammonium (DM12),
trimethylstearyl ammonium (MC18),
trimethylhexadecyl ammonium (MC16),
dimethyldecyltetradecyl ammonium (C10C14),
dimethyldidecyl ammonium (DC10),
dimethyldihexacosanyl ammonium (DC26),
dimethyltetradecyl hexacosanyl ammonium (C14C26), and
dimethylhexadecyl tetraeicosanyl ammonium (C16C24), were also prepared in a similar manner.

According to elemental analysis, among them,
dimethyldihexacosanyl ammonium (DC26),
dimethyltetradecyl hexacosanyl ammonium (C14C26), and
dimethylhexadecyl tetraeicosanyl ammonium (C16C24),
respectively were not formed in a heparin complex .

### Experiment 1

The heparin complexes were evaluated for their solubility in organic solvents shown in Table 1 at the concentration of 0.1% by weight. The results are shown in Table 1.

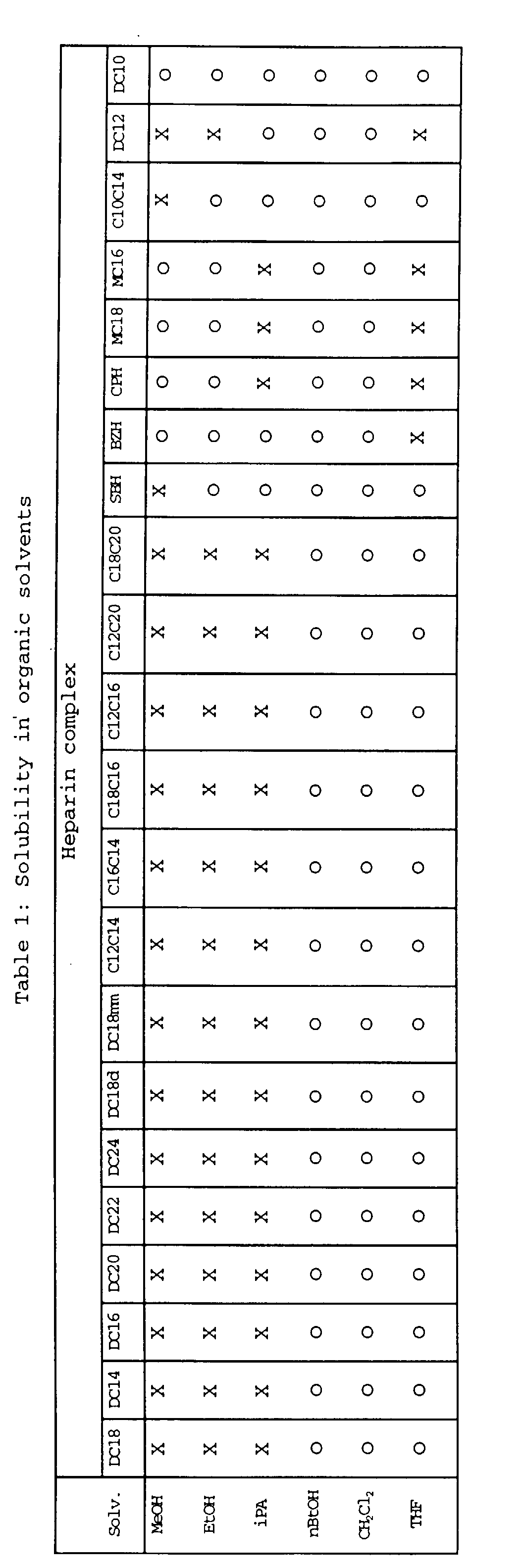

In Table 1, above,
(MeOH) : methanol having a dielectric constant of 32.65;
(EtOH) : ethanol having a dielectric constant of 24.3 ;
(iPA) : isopropanol having a dielectric constant of 18.3 ;
(nBtOH) : n-butanol having a dielectric constant of 17.1;
(CH₂Cl₂) : methylene chloride having a dielectric constant of 9.08; and
(THF) : THF having a dielectric constant of 7.39.

As shown in the results, the heparin complex of the present invention is highly hydrophobic and hardly soluble in water, and therefore, the medical device coated with such a heparin complex exhibits a reduced dissolution of the heparin complex coated on its surface in physiological saline or blood.

### Experiment 2

The heparin complexes of the Example and of the Comparative Example were compared for their solubility in an imitation blood by using physiological saline containing 10% by weight of a nonionic surfactant (Triton X-405) for the pseudo blood. The results are shown in Table 2.

**Table 2 : Solubility in imitation blood**

| Concentration | Heparin complex | | | | | | | | |
|---|---|---|---|---|---|---|---|---|---|
| | DC18 | DC14 | C18C16 | SBH | BZH | CPH | MC18 | MC16 | DC12 |
| 4 % | X | X | X | O | O | O | O | O | X |
| 10 % | X | X | X | O | O | O | O | O | X |
| 20 % | X | X | X | O | O | O | O | O | Δ |

As shown in the results, the heparin complexes of the Comparative Example are highly soluble in both blood and physiological saline. When such substance is used for the coating of an extracorporeal circulation medical system such as a supplementary oxygenator system or a medical device such as a catheter, there would be a fair risk of the dissolution of a considerable amount of the heparin complex into the blood or physiological saline used for priming purposes, and in such a case, an anticoagulation function of the substance would not actually be accomplished. If such a function of the anticoagulant substance is to be accomplished, use of the substances in a large amount would be necessary. In contrast, when the heparin complexes of the present invention are used for the coating of the medical device, the anticoagulation function of the substances is fully accomplished since there is no risk of dissolution of the substance into blood or physiological saline.

### Experiment 3

The heparin complexes of the Example were dissolved in a mixed solvent of isopropanol/THF (9:1) to 0.2% by weight. A polyvinyl chloride sheet was coated with the heparin complex by dipping the sheet into the resulting solution. The heparin complexes of the Comparative Example were also dissolved in THF to 0.2% by weight, and used for the coating of the polyvinyl chloride sheet.

The surfaces of the thus coated sheets were washed with physiological saline for 4 hours, and heparin activity of the surface was measured in accordance with a standard manner using Testzym Heparin S (manufactured by Daiichi Kagaku Yakuhin) for the samples each having a surface area of 0.2 cm². The results are shown in Table 3.

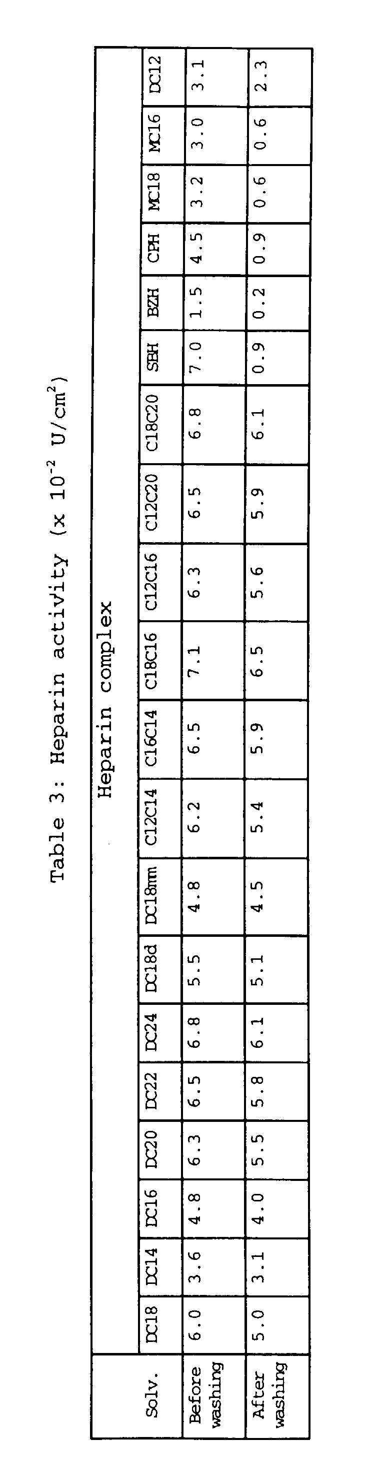

As demonstrated in the results, the heparin complexes of the present invention coated on the surface of a medical device would be capable of retaining their anticoagulation activity at a sufficient level after being kept in contact with the physiological saline or blood for a prolonged period of time.

### Experiment 4

Each heparin complex of the Example was dissolved in a mixed solvent of isopropanol/THF (7:1) to 0.2% by weight. In the resulting solution was dipped a polyvinyl chloride tube having an inner diameter of 1/4 inch and a length of 25 cm to coat the tube with the heparin complex. Opposite ends of the tube were connected with each other to form a ring-shaped test sample. The test sample was charged with 2.5 ml of bovine plasma having citric acid added thereto, and the test sample was rotated at 8 rpm. At 10 minutes from the start, the bovine plasma in the test sample was replaced with 2.5 ml of the fresh plasma. The collected bovine plasma samples were evaluated for the amount of heparin dissolved therein by using Testzym Heparin S (manufactured by Daiichi Kagaku Yakuhin). After 6 hours from the last charge of the test sample tube, the bovine plasma was removed, and the interior of the test tube sample was washed with physiological saline to measure the heparin activity remaining in the interior surface of the tube sample. A similar test was conducted for the heparin complex of the Comparative Example. The results are shown in Table 4.

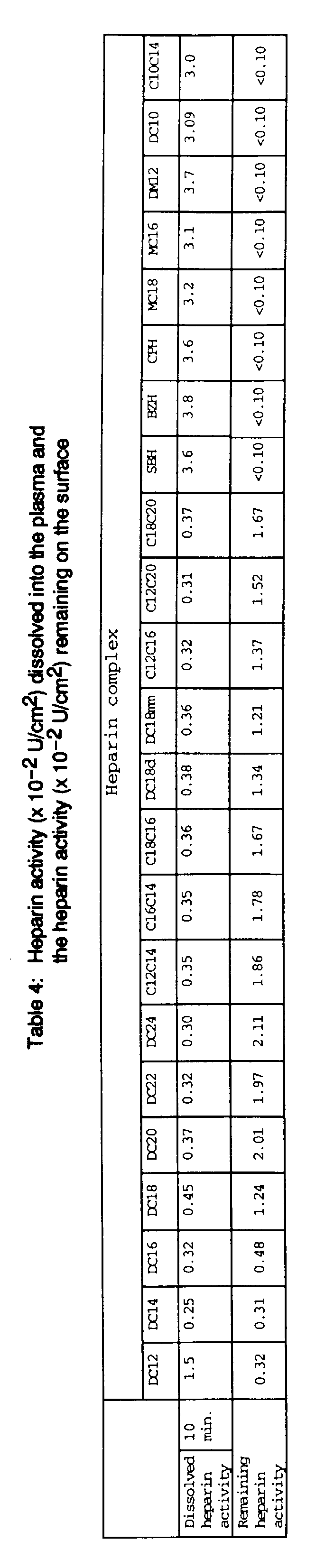

As demonstrated in Table 4, when the heparin complex of the present invention is coated on a medical device and the coated surface is kept in contact with the physiological saline or blood for a prolonged period of time, the heparin complex that dissolves into the solution would be quite little and the coated surface of the medical device will retain sufficient anticoagulation properties.

### EFFECTS OF THE INVENTION

The heparin complex of the present invention exhibits a markedly reduced dissolution into the physiological saline or blood when it is coated on a surface of a medical device and the surface is kept in contact with such solution for a prolonged period of time. Therefore, the surface of the medical device coated with the heparin complex of the present invention will retain high anticoagulation properties for a prolonged period. The heparin complex of the present invention may be effectively used for various medical devices that are brought into contact with physiological saline and blood including catheters, blood circuits, dialyzers, artificial (supplementary) hearts, oxygenators, dwelling needles, artificial kidneys, stents, and the like.

## Claims

1. A heparin complex comprising a cationic alkylammonium and heparin and does not substantially dissolve in blood.

2. A heparin complex comprising a cationic alkyldimethylammonium represented by formula (1): 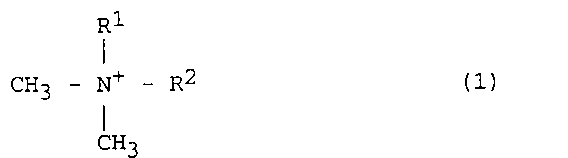 wherein R¹ and R² are independently an alkyl or an alkenyl group, when R¹ and R² are the same, R¹ and R² represent any alkyl or alkenyl group containing 12 to 24 carbon atoms, and when R¹ and R² are not the same, each R¹ and R² represents any alkyl or alkenyl group containing at least 10 carbon atoms and the sum of the carbon atoms in R¹ and R² is 26-38; and heparin.

3. The heparin complex according to claim 2 wherein said cationic alkylammonium is dimethyl didodecyl ammonium, dimethyl ditetradecyl ammonium, dimethyl dihexadecyl ammonium or dimethyl dioctadecyl ammonium.

4. A medical device wherein at least a part of the medical device has said heparin complex according to any one of claims 1 to 3 on its surface.
